## Europäisches Patentamt
## European Patent Office
## Office européen des brevets

(11) Publication number: **0 060 452**
**B1**

(12) # EUROPEAN PATENT SPECIFICATION

(45) Date of publication of patent specification: **23.10.85**

(51) Int. Cl.⁴: **A 61 N 1/30**, A 61 N 1/24

(21) Application number: **82101694.6**

(22) Date of filing: **04.03.82**

(54) Iontophoretic device.

(30) Priority: **05.03.81 US 240740**
**06.03.81 US 241284**

(43) Date of publication of application:
**22.09.82 Bulletin 82/38**

(45) Publication of the grant of the patent:
**23.10.85 Bulletin 85/43**

(84) Designated Contracting States:
**CH DE FR GB IT LI NL SE**

(56) References cited:
**CH-A- 424 006**
**DE-A-2 558 525**
**DE-B-2 147 704**
**FR-A-2 307 554**
**FR-A-2 336 144**
**FR-A-2 351 670**
**GB-A- 410 009**
**GB-A-2 030 453**
**GB-A-2 063 072**
**US-A-3 025 858**
**US-A-3 122 137**
**US-A-4 082 087**

(73) Proprietor: **MEDTRONIC, INC.**
**3055 Old Highway Eight**
**Minneapolis Minnesota 55440 (US)**

(72) Inventor: **Spevak, Richard P.**
**2619 Ardan Ave.**
**Mounds View, MN 55432 (US)**
Inventor: **Lattin, Gary A.**
**6823 145th Avenue NE**
**Forest Lake, MN 55025 (US)**
Inventor: **Jevne, Allan H.**
**1314 153rd Lane NE**
**Anoka, MN 55303 (US)**

(74) Representative: **Schwan, Gerhard, Dipl.-Ing.**
**Elfenstrasse 32**
**D-8000 München 83 (DE)**

Courier Press, Leamington Spa, England.

## Description

This invention relates to a device for use in iontophoretically introducing an ionic substance into body tissue, comprising a casing containing an electrical current source and active electrode means mounted on said casing and electrically coupled to said current source, said active electrode means including first electrode plate means located on said casing for mounting on said casing an electrode element containing said ionic substance and for electrically coupling said electrode element to said current source; and further comprising indifferent electrode means electrically coupled to said current source.

Iontophoresis is a method for introducing ionic substances into a body. The method utilizes direct electrical current to drive the ionized substances, such as chemicals or drugs, through the intact skin or other body surface. This has proven to be very useful in numerous medical applications (US—A—3 991 755 and US—A—4 141 359). Prior art iontophoretic devices generally comprise an iontophoretic current generator and a pair of electrodes. The electrode containing the ionic substance to be introduced into the body is generally known as the active electrode while the other electrode is called the ground or indifferent electrode. The current generator is generally a hand-held or table-supported instrument and the electrodes are connected to the generator by means of wires. The electrodes, especially the active electrodes, generally are relatively complex devices, including a cup or other receptacle for holding a fluid solution of the ionic substance in contact with the body, a means for making electrical contact with the solution, and a means for holding the electrode firmly to the body. Such electronic devices also require the use of some means for introducing the solution containing the ionic substance into the electrode receptacle, such as a syringe. Relatively high skill levels are thus required for the use of the prior art iontophoretic devices (compared for example to the skill required to apply a bandage). These devices also require the patient to remain in a fixed location during iontophoretic processes, which is a decided disadvantage since the process generally takes time on the order of minutes or sometimes, hours.

US—A—4 141 359 discloses a compact iontophoretic device in the embodiment shown in Fig. 2 and discussed in Example I of that patent. In this device the grounding electrode is mounted on the current generator housing and the indifferent electrode is connected to the housing by a wire. This device may be entirely Mounted on the body of the patient and thus, presumably some motion of the patient is possible while the device is in use. However, the use of this device still requires a relatively high level of skill and care since the electrodes are of the fluid gel-type. In addition, the gel-type electrodes are subject to some movement on the skin, and the wire can be snagged during movement. Thus, this device does not readily lend itself to ambulatory type applications, especially in the case of young children who tend to have highly mobile limbs.

Furthermore a device for iontophoretic treatment of the eye is known from US—A—3 122 137. This device comprises a casing containing an electrical current source, an active electrode mounted on the casing and electrically coupled to the current source, and an indifferent electrode likewise electrically coupled to the current source. The active electrode includes electrode plate means located on the casing for mounting on the casing an electrode element containing the ionic substance and for electrically coupling the electrode element to the current source. The indifferent electrode either is positioned remote from the casing and is coupled to the current source by a wire, or is defined by the conductive casing itself.

In the prior art the polarity of the current source, and thus of the electrodes is fixed. Thus, for a given ionicity, one of the electrodes is always the active electrode, while the other electrode is always the indifferent electrode. The active electrode includes a source of the ionic substance, while the indifferent electrode does not. In these prior art devices only one of the electrodes provides a source of the ionic substance. Further, if the electrodes are accidently connected to the wrong terminals of the current source the ionic substance will not be iontophoresed. Further, in many such devices, if it is discovered that the electrodes have been reversed the device must be removed from the body, the electrodes replaced correctly and then the device must be reapplied.

An object of the invention is to provide a device for iontophoretically introducing an ionic substance into body tissue in which the entire device, including the electrodes, form a single compact unit, and which allows easy mounting of a disposable electrode element.

According to the invention a device for use in iontophoretically introducing an ionic substance into body tissue is characterized in that said casing is insulating; that said indifferent electrode means likewise is mounted on said casing and includes second plate means located on said casing for mounting on said casing a second electrode element and for electrically coupling said second element to said electric current source; and that said electrode elements are removably provided on said plate means in the form of adhesive strips.

Preferably the plates are composed of stainless steel, and preferably means are provided for switching the polarity of the electrodes.

The device of the present invention simplifies the iontophoretic administration of ionic substances. The casing and plate combination forms an integral unit that can survive considerable abuse and requires little care. The device may be utilized by applying adhesive electrode pads to the metal plates and then applying the entire unit to the body in a manner similar to the application of a self-adhesive bandage. Thus this iontophoretic device lends itself to rapid use and reuse that, for the first time, makes iontophoretic devices available for ambulatory therapeutic and diagnostic uses.

A safety strap may be fixed to the casing for further securing the device to the body; the safety strap is particularly useful when the electronics of the current source are relatively bulky, but as the electronics are reduced, as for example when custom microelectronic circuits and power sources are used, the safety strap may be eliminated.

Numerous other features, objects and advantages of the invention will become apparent from the following detailed description when read in conjunction with the accompanying drawing.

In the drawing:

Fig. 1 is a partially cut away side view of a preferred embodiment of an iontophoretic device according to the invention showing one adhesive electrode pad in place on the device;

Fig. 2A is an end view of an adhesive electrode as it may be manufactured and sold;

Fig. 2B shows the electrode pad of Fig. 2A just prior to its application to a preferred embodiment of the invention;

Fig. 3 is a bottom view of the device of Fig. 1;

Fig. 4 is a top view of the device of Fig. 1;

Fig. 5 is a block diagrammatic illustration of circuitry for an iontophoretic device;

Fig. 6 is a block diagrammatic illustration of the preferred electronic circuitry for producing the iontophoretic current;

Fig. 7 is a more detailed diagrammatic illustration of the electronic circuity of Fig. 6;

Fig. 8 is a block diagrammatic illustration of an alternative embodiment of an iontophoretic system according to the invention;

Fig. 9 is a partially cut-away side view of an iontophoretic device according to the preferred embodiment of the invention diagrammed in Fig. 8;

Fig. 10 is a top view of the device of Fig. 9;

Fig. 11 is a block diagrammatic illustration of the electronic circuitry of the alternative preferred embodiment of the invention;

Fig. 12 is a more detailed electronic diagram of the circuitry according to a preferred embodiment of the alternative embodiment of the invention; and

Fig. 13 is a detailed electronic diagram of another alternative preferred embodiment of the invention.

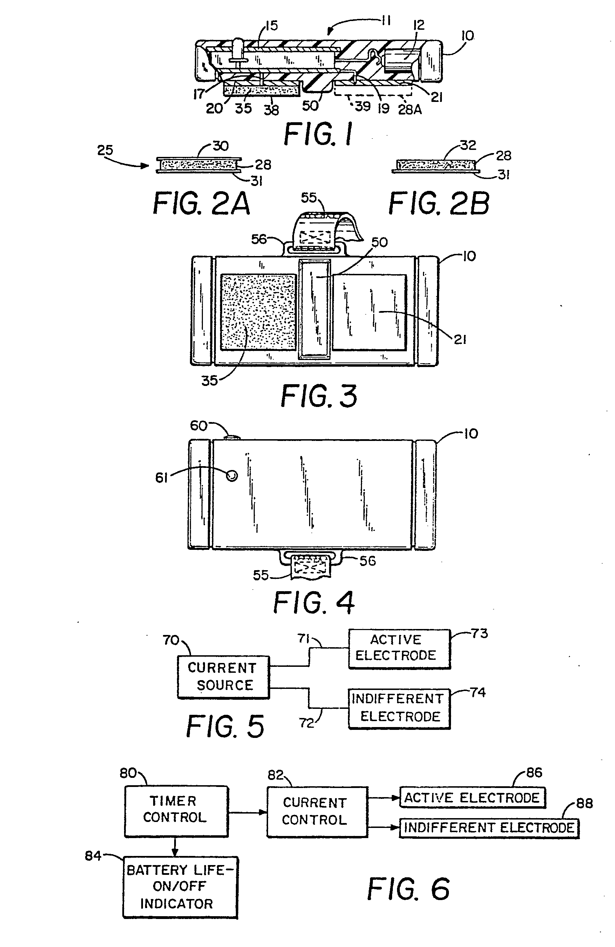

A cut away view of an exemplary embodiment of the invention is shown in Fig. 1. Casing 10 contains electric current source 11 which includes a battery power source 12 and an electronics package 15. The electric current source 11 is coupled via leads 17 and 19 to electrode plate means 20 and 21 respectively.

The embodiment of the invention shown in Fig. 1 may be prepared for use by applying electrode elements, such as shown in Figs. 2A and 2B to electrode plates 20 and 21. The electrode 25 comprises a sheet-like adhesive pad 28 having protective backing sheets 30 and 31 covering its broad surfaces as shown in Fig. 2A. In Fig. 2B the protective sheet 30 has been removed exposing the adhesive surface 32. The pad 28 may be then applied to one of the electrode plates of the device such as 21. In Fig. 1 the pad is shown in ghost at 28A in the position it would occupy when applied to plate 21. The entire device may then be applied to the body. It is understood that "body" is used in its most general sense and includes plant, animal and human bodies.

Adhesive pads such as 35 and 28 are composed of an ionic substance admixed with an adhesive. When the device of Fig. 1 is applied on the body with the lower surfaces such as 38 of the pads against the body and the device is turned on, the ionic substance in one or both of the pads is driven into the body. For example, if the ionic substance that is desired to drive into the body is a positive ionic substance, a pad containing this substance may be placed on the positive electrode plate 20 and an indifferent electrode pad in which the ionic substance may be a salt such as sodium chloride, which has ions of both positive and negative polarity, may be placed on the negative electrode. The electrode pads such as 28 and 35 and the backing sheets, such as 30 and 31, may be color-coded for identification and/or may be marked with positive or negative symbols to clearly identify which of plates 20 and 21 they should be applied to. The adhesive pads such as 28 and 35 are more fully discussed in European Patent Application Serial No. 82 101 692 0.

Turning attention to Fig. 3 a bottom view of the device according to the invention is shown. It is seen that in this embodiment the plates 20 (shown covered with pad 35 in Fig. 3) and 21 are of generally square shape, although any shape may be chosen. A negative sign and a positive sign (not illustrated) can be provided on plate 21 and on plate 20, respectively. Plates 20 and 21 are separated by a raised ridge 50, portion of housing 10, which ridge 50 ensures the electrical separation of pads 35 and 28A. As can be seen in Fig. 1, ridge 50 extends below the surface of plates 20 and 21 but does not extend as far as the surfaces 38 and 39 of pads 35 and 28A. Should the iontophoretic device be attached to the limb or other portion of the body with such force that pads 35 and 28 become compressed, or if pads 35 and 28A should become displaced in a horizontal direction from their correct position, ridge 50 prevents their physical contact which would short out the iontophoretic circuit. Ridge 50 also assists in the rapid application of pads such as 35 and 28A to plates 20 and 21.

Strap 55 is provided to assist in holding device 11 to the body to which the device is applied. Strap 55 is attached to strap anchor 56 on housing 10. In the embodiment shown, strap 55 is a material which adheres to itself, such as VELCRO[RTM]. It attaches to itself after circling the limb or other body portion, and thus only one strap anchor 56 is necessary. Strap 55 is provided because in the embodiment shown, housing 10, battery 12 and electronics 15 having sufficient mass to that the device might become separated from the body during rapid movements of the limb or other body portion to which it is applied. The invention contemplates that circuitry 15 and power source 12 may be made extremely small and of relatively low mass using state of the art technology, in which case strap 55 may be eliminated.

As shown in Fig. 4, OFF/ON switch 60 and light-emitting diode (L.E.D.) 61 protrude through housing 10. Switch 60 is used to activate the iontophoretic device. Diode 61 serves to indicate whether the current is on or off and provides battery life indication. These shall be discussed below in more detail in connection with the electronic circuitry.

Turning to Fig. 5 there is shown a block diagrammatic illustration of the iontophoretic circuit. This circuit includes current source 70 which is electrically coupled through coupling means 71 and 72 to electrodes 73 and 74. For purposes of illustration, electrode 73 is labeled the active electrode while electrode 74 is labeled the indifferent electrode, although the positions could be reversed. Coupling means 71 and 72 may be wires or any other means for electrically coupling the current source and the electrodes.

Fig. 6 shows a more detailed block diagrammatic illustration of the electrical circuit employed in the embodiment of the invention disclosed in reference to Figs. 1—4. Turning he switch 60 (not shown in Fig. 6) on activates timer control circuit 80. Timer control circuit 80 provides a signal to battery life—ON/OFF indicator circuit 84 which, in turn, activates light-emitting diode 61 provided that the battery voltage is above a predetermined level which is considered to be sufficient to reliably operate the device. Timer control circuit 80 also provides a signal to current control circuit 82. Current control circuit 82 responds to a signal from timer control circuit 80 to ramp/on the iontophoretic current; that is, the iontophoretic current is turned on gradually from 0 value up to the full current value. This prevents burning, shocking or other unpleasant sensations when the current is turned on. The iontophoretic current is applied from current control circuit 82 to electrodes 86 and 88. In the embodiment shown the current is such that it flows from active electrode 86 through the body to indifferent electrode 88; in other embodiments the electrodes or the direction of the current may be reversed.

The dosage of ionic substance which is applied to the body is controlled by timer control circuit 80 and current control circuit 82. Current control circuit 82 provides a constant current output to the electrode which is, within impedance range and as limited by supply voltage, independent of the load between electrodes 86 and 88, which is generally the skin impedance. Thus, the amount of ionic substance driven into the body by the current will be constant in time. In this manner control of the time over which the current is applied controls the dosage. After a predetermined amount of time timer control circuit 80 applies a second signal to current control circuit 82 which causes current control circuit 82 to turn the iontophoretic current off. At the same time a second signal is passed through battery life—ON/OFF indicator circuit 84 which causes the circuit to turn L.E.D. 61 off.

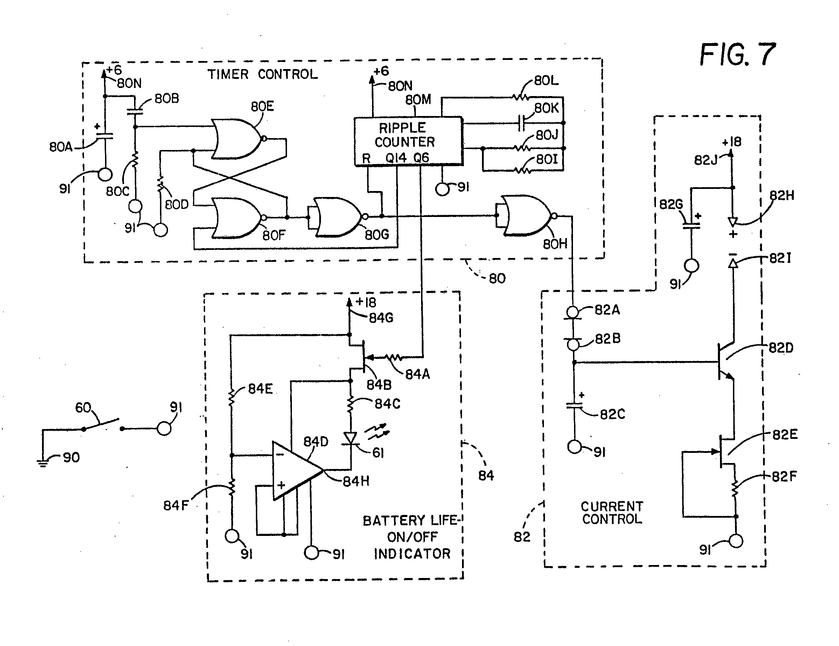

The electroncs for the circuit of Fig. 6 is shown in detail in Fig. 7. Switch 60 connects ground 90 and an input line connecting to each of the subcircuits.

Timer control circuit 80 comprises .1 microfarad capacitor 80B, 1 megohm resistor 80C and 80D, NOR gates 80E through 80H, 910 kilohm resistor 80I, 560 kilohm resistor 80J, .056 microfarad capacitor 80K, 3.3 megohm resistor 80L, and ripple counter 80M.

As is well known in the literature, a NOR gate is an electronic device with one output and two or more inputs. If either of the inputs to a NOR gate is the positive circuit voltage (6 volts in the case of the present circuit), conventionally referred to as the logic "1" state, then the output of the NOR gate is the ground voltage, conventionally referred to as the logic "0" voltage. If all the inputs are in the logic "0" state, then the signal at the output is a logic "1" state. The conventional provisions of power supply voltages and ground voltages to the NOR gates are not shown.

Capacitor 80A is connected between switch 60 and the positive 6 volt voltage terminal 80N. Capacitor 80A is a 33 microfarad filter capacitor whose purpose is to reduce circuit noise. Capacitor 80B is connected between the high voltage side of capacitor 80A and the upper input terminal to NOR gate 80E, which terminal is also connected to switch 60 through resistor 80C. NOR gates 80E and 80F are cross-coupled in a standard configuration for a flip-flop circuit. The output of NOR gate 80E is connected to the upper input to NOR gate 80F. The lower input to gate 80F is connected to the Q14 counter (No. 3 pin) output of ripple counter 80M, the output of NOR gate 80F is connected to the two inputs of gate 80G and to the lower input of gate 80e, the latter input also being connected to switch 60 through resistor 80D. The output of NOR gate 80G is connected to the RESET input (No. 12 pin) of the ripple counter 80M and also to both inputs of NOR gate 80H. Resistors 80I and 80J are connected in parallel between the No. 10 pin of ripple counter 80M and one side of capacitor 80K, which is also connected through resistor 80L to the No. 11 pin of ripple counter 80M. The other side of capacitor 80K is connected to the No. 9 pin of ripple counter 80M. The No. 8 pin of ripple counter 80M is connected to ground through switch 60 while the No. 16 pin is connected to the +6 volt supply 80N. Ripple counter 80M is a CD 4060 ripple counter divider available from RCA Solid State Division, Box 3200, Somerville, NJ, 08876. The output of NOR gate 80H is applied to the current control circuit which will be described below.

When switch 60 is closed the inputs to NOR gate 80G will be at a logic "0" since they are connected to ground through resistor 80D, and no current is initially flowing in the line. The output of gate 80G will thus be a logic "1" which resets ripple counter 80M setting the Q14 output to a logic "0", and thus the lower input to gate 80F to a logic "0". At the same time, a current will begin flowing in the circuit from positive terminal 80N through resistor 80C to ground (charge will be building on capacitor 80B) which will set the upper input terminal to NOR gate 80E at a logic "1". The output of gate 80E is thus forced to a logic "0" which is applied to the upper input of gate 80F. The two inputs to gate 80F being a logic "0", the output will switch to a logic "1". This causes the output of gate 80G to go to a logic "0", which releases the RESET on ripple counter 80M which permits it to begin counting and at the same time causes the output of gate 80H to switch from a logic "0" to a logic "1". While ripple counter 80M is counting the upper input to NOR gate 80E falls to a logic "0", however the output of the gate is maintained to a logic "0" because the lower input to the gate is held at a logic "1" as long as the lower input of gate 80F is held to logic "0".

Ripple counter 80M is driven by an oscillator circuit consisting of resistors 80I, 80J, 80L and capacitor 80K. The value of resistor 80I is selected so that the oscillation period is 36.6 milliseconds. The ripple counter will count the 36.6 millisecond oscillations so that within approximately 5 minutes, its 14th counter is triggered and the Q14 pin goes to a logic "1". This logic "1" signal applied to the lower input of gate 80F causes the output of the gate to go to logic "0", which in turn forces the output of gate 80G to a logic "1" which holds ripple counter 80M RESET and changes the output of NOR gate 80H to a logic "0". The logic "0" output of NOR gate 80F is also applied to the lower input of NOR gate 80E. Both inputs of NOR gate 80E being a logic "0", the output will become a logic "1", which is applied to upper input of NOR gate 80F forcing its input to a logic "0", thereby latching gates 80E and 80F in a state such that the output of NOR gate 80H remains a logic "0" until switch 60 is turned off and then on again to restart the cycle. Thus, timer control circuit 80 produces a logic "1" signal to current control circuit 82 for a five-minute period after switch 60 is turned on. While counter 80M is counting up to five minutes, its 6th counter stage will go to a logic "1" approximately every 2.3 seconds. The output of the 6th counter stage (No. 4 pin) will thus go to a logic "1" for a 1.15 second period every 2.3 seconds. This signal is passed to the battery life—ON/OFF indicator circuit 84 and used as discussed below.

Current control circuit 82 includes two constant current 1N 5290 diodes 82A and 82B, 330 microfarad capacitor 82C, a 2N 2222 transistor 82D, a 2N 4341 FET 82E, 510 kilohm resistor 82F, 33 microfarad capacitor 82G and electrodes outputs 82H and 82I.

The anode of constant current diode 82A is connected to the output of NOR gate 80H in timer control circuit 80. The cathode of diode 82A is connected to the cathode of diode 82B while the anode of diode 82B is connected to one side of capacitor 82C and also to the base of transistor 82D. The other side of capacitor 82C is connected to ground through switch 60. The emitter of transistor 82D is connected to the drain of FET 82E. The gate of FET 82E is connected to switch 60 and is also connected to its own source through resistor 82F. The collector of transistor 82D is connected to the negative electrode output 82I which may be connected to plate 21 in the embodiment of the invention shown in Figs. 1, 3 and 4. The other electrode output 82H, which may be connected to plate 20 in the embodiment shown in Figs. 1, 3 and 4, is connected to the positive 18 volt power source 82J, and the line between electrode 82H and power source 82J is connected to one side of capacitor 82G. The other side of capacitor 82G is connected to switch 60.

Capacitor 82G is a filter capacitor to eliminate noise in the circuit. When timer control circuit 80 causes NOR gate 80H to go to a logic "1" state immediately after switch 60 is turned on, a 6 volt signal is applied to the anode of diode 82A. Since this is a constant current diode the current that passes through it is fixed and thus the charge on capacitor 82C is built up slowly, over a period of about 1 second. Thus, the voltage applied to the base of transistor 82A builds up slowly, to the full 6 volt value over the same period, causing the transistor to turn on slowly over the same period. FET 82E and resistor 82F form a conventional current-limiting circuit. Resistor 82F is chosen to limit the current to 2 milliamps. A circuit is completed through the body and through electrode output 82H to the positive voltage source. Thus, upon application of the signal from the timer control circuit 80, the current control circuit 82 slowly, over a period of about a second, ramps up the current through the electrode outputs 82I and 82H from 0 to a maximum constant current of 2 milliamps. When, at the end of the 5-minute period, the signal from timer control circuit 80 drops to a logic "0" the charge on capacitor 82C will slowly drain through current-limiting diode 82B, again over about a 1-second period. Thus, the transistor 82D will be slowly turned off over the same period and the current through the electrodes will slowly ramp down to a 0 value.

Battery life—ON/OFF indicator circuit 84 comprises 100 kilohm resistor 84A, 2N4338 FET 84B, 1.5 kilohm resistor 84C, L.E.D. 61, an LM10H differential amplifier 84D, which is available from National Semiconductor Corp. at 2900 Semiconductor Drive, Santa Clara, CA 95051, 820 kilohm resistor 84E and 12 kilohm resistor 84F. The gate of FET 84B is connected to the Q6 output (No. 4 pin) of decade counter 80M in timer control circuit 80 through resistor 84A.

The drain of FET 84B is connected to the positive 18 volt power source 84G and to switch 60 through resistor 84E and 84F. The source of FET 84B is connected to the output 84H (No. 6 pin) of amplifier 84D through resistor 84C and L.E.D. 61, and is also connected to the No. 7 pin of amplifier 84D. The negative input terminal (No. 2 pin) of amplifier 84D is connected to the line between resistors 84F and 84E. The positive input terminal (pin No. 3) of amplifier 84D is connected to both the No. 1 and No. 8 pins of the same amplifier. The No. 4 input pin of the amplifier 84E is connected to switch 60.

As discussed above, the Q6 output of ripple counter 80M in timer control circuit 80 will go to a logic "1" for a 1.15 second period once each 2.3 seconds while the counter is running. Each time it goes to a logic "1" FET 84B is turned on for the 1.15 second period. The circuit consisting of amplifier 84D, resistors 84C, 84E, and 84F and L.E.D. 61 is a conventional battery-level test circuit disclosed in the applications manual for the LM10H amplifier published by National Semiconductor Corporation. When FET 84B turns on it activates amplifier 84D which compares the voltages between its negative and positive inputs. If the battery charge is higher than 14 volts the amplifier will connect its No. 6 pin output terminal 84H to ground 90. If switch 60 is closed this will close the circuit from the positive terminal 84G to ground 90 through L.E.D. 61, causing the L.E.D. to operate. If the battery voltage is below 14 volts, amplifier 84D will not connect output 84H to ground and L.E.D. 61 will not turn on. Thus, battery life—ON/OFF indicator circuit 84 will cause L.E.D. 61 to blink at 2.3 second intervals during the five-minute period when the current is on, providing the battery level is above 14 volts.

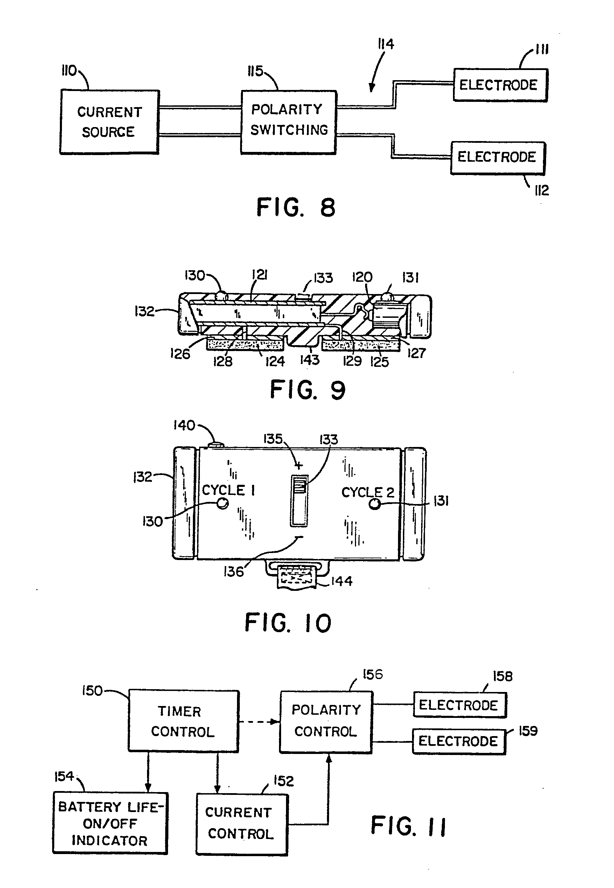

A block diagrammatic illustration of an alternative embodiment of the invention is shown in Fig. 8. Current source 110 produces an electric current which is delivered to electrodes 111 and 112 by electrical connection means 114. A polarity switching means 115 is interposed between the current source and the electrodes and controls their polarity. For example, the polarity may be such that electrode 111 is positively charged and electrode 112 is negatively charged, or the polarity may be reversed so that electrode 111 is negatively charged and electrode 112 is positively charged.

Figs. 9 and 10 show a partially cut-away side view and a top view respectively of an exemplary embodiment of the above alternative embodiment of the invention. In this embodiment current source 110 comprises a battery pack 120 and a circuit package 121 for generating and controlling the current. In this embodiment the electrodes 124 and 125 are again adhesive pads which contain the ionic substance to be driven into the body. Preferably the pads 124 and 125 comrise an adhesive substance with the ionic substance in admixture therewith. The means for connecting the electrodes 124 and 125 to the current source comprise metal plates 126 and 127, to which the adhesive electrodes 124 and 125 are applied, and leads 128 and 129 which connect the plates to the current generating electronics package 121. Light emitting diodes (LEDs) 130 and 131 which are connected to electronics circuit package 121 and which are visible externally of the casing 132 provide means for indicating which of the electrodes is delivering the ionic substance. Ion switch 133 is electrically connected to circuitry 121 and provides a means for correlating the indicating LEDs 130 and 131 with the electric charge type of the ionic substance. If an ionic substance with a positive charge is to be driven into the tissue with the device then switch 133 is placed in the position towards the plus sign 135. If an ionic substance of a negative charge is to be used, the switch 133 is placed in the direction of negative sign 136. Switch 140 is a ON/OFF switch which is electrically connected to electronic circuitry 121 and activates the iontophoretic device. Projection 143 (Fig. 9) is formed in the center bottom portion of casing 132 to assist in electrically separating electrode pads 124 and 125. Strap 144 (Fig. 10) is provided to assist in attaching the device to a portion of a body to which the device is to be applied, as for example an arm or a leg of a human being.

The device shown in Figs. 9 and 10 is used by applying the device to the body surface into which it is desired to introduce the ionic substance contained in electrodes 124 and 125. Electrodes 124 and 125 are adhesive and thus will adhere to a body surface, such as skin, holding the device in place. Strap 144 may be used to assist in holding the device in place. If a positive ionic substance has been selected for the iontophoresis process, switch 133 will have been placed in the positive position. When the device is in position, switch 140 is triggered to turn on the device. If the battery has sufficient voltage to operate the device, LED 130 will beging blinking to indicate that the device is operating properly and that electrode 124 beneath LED 130 is delivering the ionic substance. After a preselected time, the device will automatically turn down the current, switch polarity and turn up the current again. LED 131 will then begin blinking to indiciate both that the battery life is still sufficient and that the electrode 125 under light 131 is now delivering the ionic substance. After another preselected time the device will shut itself off and light 131 will go out. The time for the switching of polarity and the shutoff time is determined by dosage considerations and the time necessary to deplete the amount of ionic substance stored in the electrodes 124 and 125. Other treatment considerations such as the desire to provide a small dosage over a long period, etc may also impact on the times. In the embodiment shown, LEDs 130 and 131 also indicate whether the device is on the first or the second polarity cycle. It also should be noted that in this embodiment switch 133 also provides a means for manually switching the polarity of the electrodes if it is so desired. In addition, in this embodiment the combination of switch 133 and LEDs 130 and 131 also provide a means for indicating the polarity of the electrodes. That is, if switch 133 is in the plus position when the unit is turned on, then the light that is flashing indicates that the electrode is a positive electrode; and on the other hand if switch 133 is in the negative position when the unit is turned on, then the light flashes above the negative electrode. The current is turned down prior to the switching of the polarity, then turned up afterwards in order to prevent the unpleasant sensation to the patient which might be caused by the switching of the polarities while the device is operating at maximum current and at the same time it provides an electronic safety feature.

Fig. 11 shows a block diagrammatic illustration of an exemplary preferred embodiment of the electronics of the alternative embodiment of the invention. The electronics includes a timing control circuit 150, a current control circuit 152, a polarity control circuit 156, a battery life—ON/OFF indicator circuit 154, and electrodes 158 and 159. The turning on of the ON/OFF switch 146 (not shown in Fig. 11) activates circuits 150, 152, 154 and 156. Timer control circuit 150 provides a signal to battery life—ON/OFF indicator circuit 154 which, in turn, activates an indicating device, such as LEDs 130 and 131 in Fig. 10, provided the battery voltage is above a predetermined level which is considered to be sufficient to reliably operate the device. Timer control circuit 150 also provides a signal to current control circuit 152. Current control circuit 152 responds to the signal to ramp on the iontophoretic current; that is, the iontophoretic current is turned on gradually from a zero value up the full current value. This prevents burnings, prickings, or other unpleasant sensations when the current is turned on. The iontophoretic current produced by current control circuit 152 is applied to electrodes 158 and 159 through polarity control circuit 156. Polarity control circuit 156 determines the direction in which the current flows between electrodes 158 and 159, and thus control which of the electrodes is the active electrode. The switching of the polarity may be either manually by means of an external switch (not shown in Fig. 11) or automatically by circuit 156 in response to a signal from timing control circuit 150 as shown by the dotted arrow.

The dosage of the ionic substance which is applied to the body is controlled by timer control circuit 150 and current control circuit 152. Current control circuit 152 provides a constant current output to the electrodes 158 and 159 which is independent of the load within the power supply limits, which load is generally skin impedance. Thus, the amount of ionic substance driven into the body by the current will be constant in time. In this manner, control of the time over which of the current is applied controls the dosage. After a predetermined amount of time, timing control circuit 150 applies a second signal to current control circuit 152 which causes the current control circuit to ramp the iontophoretic current down. Again, the ramping is to prevent any unpleasant affects. Alternatively, the dosage may be controlled manually by turning off the ON/OFF switch (not shown in Fig. 11) at a chosen time.

In the embodiments in which the polarity is controlled manually, the polarity may be switched after the timing control circuit ramps down the current. In embodiments where the polarity control is automatic, a timing signal to the polarity control circuit 156 will switch the polarity automatically after the current is ramped down. After the polarity is switched, the current may be turned back on either by the manual ON/OFF switch or, in the case of the automatically controlled embodiments, by a timing signal to current control circuit 152 which causes the current to ramp up again. After a second predetermined time, a further signal from timing control circuit 150 to current control circuit 152 again causes the current to ramp down and the device to turn itself off. Signals from timing control circuit 150 to the battery life—ON/OFF indicator 154 cause the indicator LEDs to go off each time the circuit is ramped down, and in the automatic embodiments to indicate which electrode is the active electrode.

In general, it should be noted that the word ramping does not necessarily indicate that the current is turned completely off or is turned down or up with any specific rate; it refers to a turning down or up to the current in a manner that will prevent injury or unpleasantness etc.

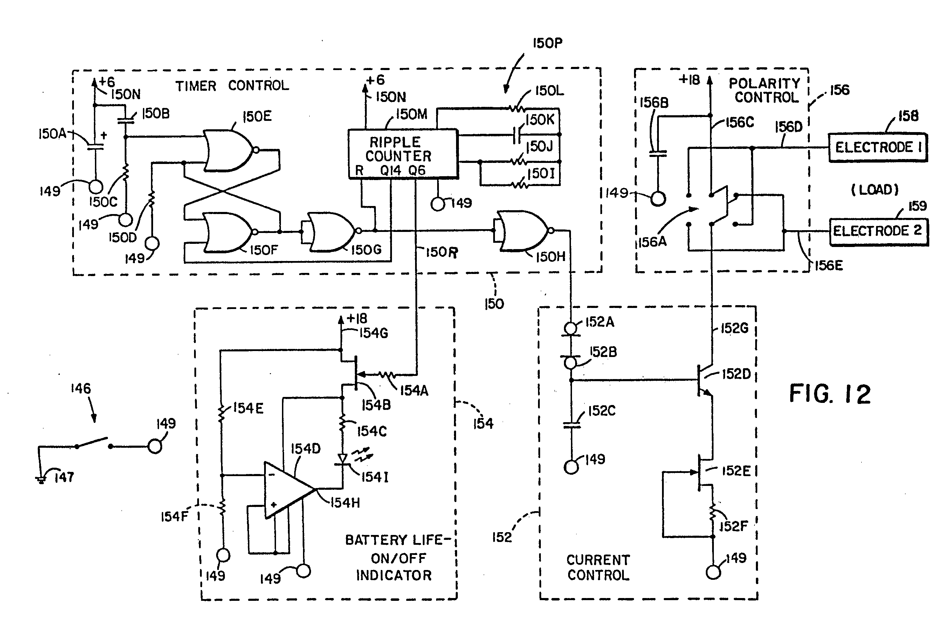

A more detailed electronic diagram of an exemplary embodiment of the alternative embodiment of the invention is shown in Fig. 12. The circuit is arranged so that the individual subcircuits 150, 152, 154 and 156 occupy approximately the same relative positions as they do in Fig. 11. ON/OFF switch 146 connects ground 147 and an input line which connects each of the subcircuits at the points indicated by an open circle, such as circle 149 indicated at the bottom of battery line—ON/OFF indicator circuit 154.

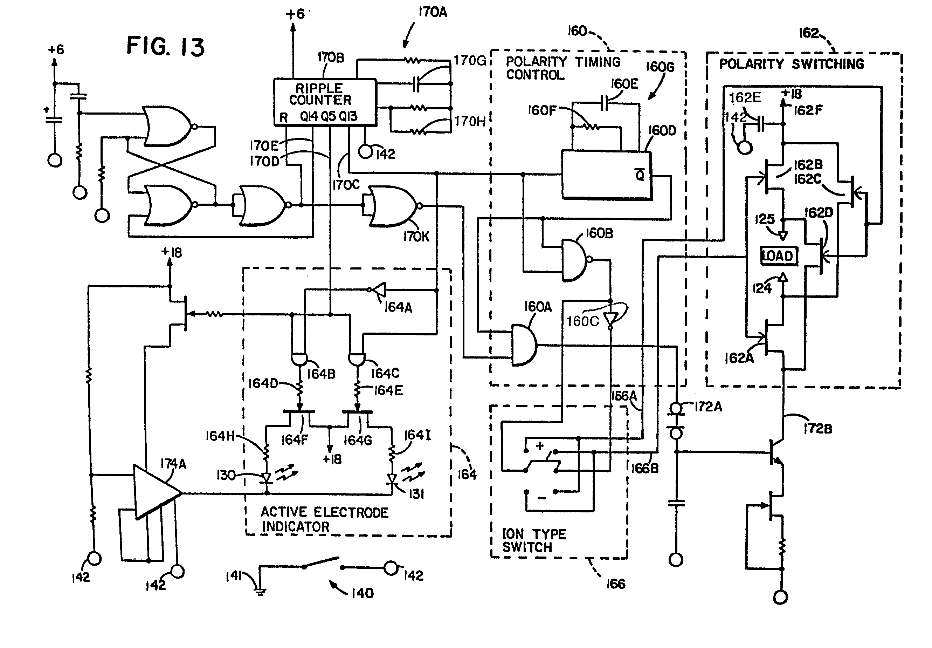

The electronic circuits of Figs. 12 and 13 include NOR gates, AND gates, a NAND gate, inverterss, and one monostable multivibrator or one-shot. These elements are shown by their conventional symbols, a NOR gate being shown at 150E in Fig. 12, an AND gate being shown at 160A in Fig. 13, the NAND gate being shown at 160B in Fig. 13, an inverter being shown at 164A in Fig. 13 and the one-shot or monostable being shown at 160D in Fig. 13. As is well known in the literature an AND gate has two or more inputs and one output. The output of an AND gate is a logic "0" signal unles the signals applied to the inputs are all a logic "1" in which case the output of the AND gate is a logic "1" signal. A NAND gate also has two or more inputs and one output. The output of a NAND gate is a logic "1" signal unless the signal applied to all of the inputs is a logic "1", in which case the output is a logic "0" signal. An inverter has one input and one output, with the output providing a signal having a logic value opposite to that of the signal applied to the input. The conventional provisions of power supply voltages and ground voltages to the gates, inverters, and flip-flop are not shown.

Timer control circuit 150 (Fig. 12) comprises 33 microfarad capacitor 150A, .1 microfarad capacitor 150B, 1 megohm resistors 150C and 150D, NOR gates 150E though 150H, 910 kilohm resistor 150I, 560 kilohm resistor 150J, .056 microfarad capacitor 150K, 3.3 megohm resistor 150L and ripple counter 150M.

Capacitor 150A is connected between the line 149 to ON/OFF switch 146 and the positive 6 volt voltage terminal 150N. This is a filter capacitor whose purpose is reduce circuit noise, and is not otherwise actively involved in the timing control function. Capacitor 150B is connected between the high voltage side of capacitor 150A and the upper input terminal to NOR gate 150E, which terminal is also connected to ground input line 149 through resistor 150C. NOR gates 150E and 150F are coupled in a standard configuration for a flip-flop circuit. That is, the output of NOR gate 150E is connected to the upper input of NOR gate 150F, while the output of NOR gate 150F is connected to the lower input to gate 150E. The lower input to gate 150F is connected to the Q14 counter (No. 3 pin) output of ripple counter 150M. The output of NOR gate 150F is also connected to the two inputs of NOR gate 150G. The lower input of gate 150E is also connected to ground input line 149 through resistor 150D. The output of NOR gate 150G is connected to the reset input (No. 12 pin) of ripple counter 150M and also to both inputs of NOR gate 150H. Resistors 150I and 150J are connected in parallel between the No. 10 pin of ripple counter 150M and one side of capacitor 150K, which is also connected through resistor 150L to the No. 11 pin of ripple counter 150M. The other side of capacitor 150K is connected to the No. 9 pin of ripple counter 150M. The No. 8 pin of ripple counter 150M is connected to ground input 149 while the No. 16 pin is connected to the +6 volt power supply 150N. Ripple counter 150M is a CD4060 ripple counter divider available from RCA Solid State Division, Box 3200, Summerville NJ 08876. The output of NOR gate 150H is applied to current control circuit 152 which will be described below.

When switch 146 is closed the inputs to NOR gate 150G will be at a logic "0" since they are connected to ground through resistor 150D, and no current is initially flowing in the line. The output of gate 150G will thus be a logic "1" which resets ripple counter 150M, setting the Q14 output to a logic "0", and thus the lower input to gate 150F to a logic "0". At the same time a current will begin flowing in the circuit from positive terminal 150N through resistor 150C to ground (charge will be building on capacitor (150B) which will set the upper terminal to NOR gate 150E at a logic "1". The output of gate 150E is thus forced to a logic "0" which is applied to the upper input of gate 150F. The two inputs to gate 150F being a logic "0", the output will switch to a logic "1". This causes the output of gate 150G to go to a logic "0", which releases the reset on ripple counter 150M, which permits it to begin counting, and at the same time causes the output of gate 150H to switch from a logic "0" to a logic "1". While ripple counter 150M is counting the upper input to NOR gate 150E falls to a logic "0", however the output of the gate is maintained at a logic "0" because the lower input to the gate is held again logic "1" as long as the lower input of gate 150F is held to a logic "0".

Ripple counter 150M is driven by oscillator circuit 150P consisting of resistors 150I, 150J, 150L and capacitor 150K. The value of resistor 150I is selected so that the oscillation period is 36.6 milliseconds. The ripple counter will count the 36.6 millisecond oscillations so that within approximately 5 minutes its fourteenth counter is triggered, and the Q14 pin goes to a logic "1". This logic "1" signal applied to the lower input of gate 150F causes the output of the gate to go to a logic "0", which in turn forces the output of gate 150G to a logic "1", which holds ripple counter 150M reset and changes the output of NOR gate 150H to a logic "0". The logic "0" output of NOR gate 150F is also applied to the lower input of NOR gate 150E. Both inputs of NOR gate 150E being a logic "0", the output will become a logic "1", which is applied to the upper input of NOR gate 150F forcing its input to a logic "0" thereby latching gates 150E and 150F in the "off" state such that the output of NOR gate 150H remains a logic "0" until switch 146 is turned off, and then on again to restart the cycle. Thus, timer control circuit 150 provides a logic "1" signal to current control circuit 152 for a 5-minute period after switch 146 is turned on. While counter 150M is counting up to five minutes, its sixth counter stage will go to a logic "1" approximately every 2.3 seconds. The output of the sixth counter stage (No. 4 pin) will thus go to a logic "1" for a 1.15 second period every 2.3 seconds. This signal is passed to battery life—ON/OFF indicator circuit 154 and used as discussed below. When the counter has fully counted the five minutes and gates 150E and 150F are latched into the "off" state all counter outputs will at a logic "0" thus holding the various subcircuits in an off position as will be further described below.

Current control circuit 152 includes two constant current 1N 5290 diodes 152A and 152B, 330 microfarad capacitor 152C, a 2N 2222 transistor 152d, a 2N 4341 FET 152E, and 510 kilohm resistor 152F.

The anode of constant current diode 152A is connected to the output of NOR gate 150H in timer control circuit 150. The cathode of diode 152A is connected to the cathode of diode 152B while the anode of diode 152B is connected to one side of capacitor 152C and also to the base of transistor 152D. The other side of capacitor 152C is connected to ground input line 149. The emitter of transistor 152D is connected to the drain of FET 152E. The gate of FET 152E is connected to ground input line 149 and is also connected to its own source through resistor 152F. The collector of transistor 152D is connected to output line 152G which is the input to polarity control circuit 156.

When timer control circuit 150 causes NOR gate 150H to go to a logic "1" state after switch 146 is turned on, a 6 volt signal is applied to the anode of diode 152A. Since this is a constant current diode the current that passes through it is fixed and thus the charge on capacitor 152C is built up slowly, over a period of about 1 second. Thus, the voltage applied to the base of transistor 152A builds up slowly, the full 6 volt value over the same period, causing the transistor to turn on slowly over the same period. FET 152E and resistor 152F form a conventional current-limiting circuit. Resistor 152F is chosen to limit the current to 2 milliamps. Thus, upon application of the signal from timer control circuit 150, current control circuit 152 slowly, over a period of about a second, ramps up the current through its output line 152G from 0 to a maximum constant current of 2 milliamps. When, at the end of the 5-minute period, the signal from timer control circuit 150 drops to a logic "0", the charge on capacitor 152C will slowly drain through current-limiting diode 152B, again over about a 1-second period. Thus, the transistor 152D will be slowly turned off over the same period and the current through the output line 152G will slowly ramp down to a 0 value.

Battery life—ON/OFF indicator circuit 154 comprises 100 kilohm resistor 154A 2N4338 FET 154B, 1.5 kilohm resistor 154C, LED 154I, an LM10H differential amplifier 154D, which is available from National Semiconductor Corp. at 2900 Semiconductor Drive, Santa Clara, CA 95051, 820 kilohm resistor 154E and 12 kilohm resistor 154F. The gate of FET 154B is connected to the Q6 output (No. 4 pin) of ripple counter 150M in timer control circuit 150 through resistor 154A. The drain of FET 154B is connected to the positive 18 volt power source 154G and to ground input line 149 through resistors 154E and 154F. The source of FET 154B is connected to the output 154H (No. 6 pin) of amplifier 154D through resistor 154C and LED 154I, and is also connected to the No. 7 pin of amplifier 154. The negative input terminal (No. 2 pin) of amplifier 154D is connected to the line between resistors 154F and 154E. The positive input terminal (No. 3 pin) of amplifier 154D is connected to both the No. 1 pin and No. 8 pin of the same amplifier. The No. 4 input pin of the amplifier 154E is connected to ground input line 149.

As discussed above, the Q6 output of ripple counter 150M in timer control circuit 150 will go to a logic "1" for a 1.15 second period once each 2.3 seconds while the counter is running. Each time it goes to a logic "1" FET 154B is turned on for the 1.15 second period provided the battery level is above the predetermined level which for this embodiment is 14 volts. The circuit consisting of amplifier 154D, resistors 154C, 154E and 154F and LED 154I is a conventional battery-level test circuit disclosed in the applications manual for the LM10H amplifier published by National Semiconductor Corporation.

When FET 154B turns on, it activates amplifier 154D which compares the voltages between its negative and positive inputs. If the battery chage is higher than 14 volts the amplifier connects its No. 6 pin output terminal 154H to ground line 149. If switch 146 is closed this will close the circuit from the positive terminal 154G to ground 147 through LED 154I, causing the LED to operate. If the battery voltage is below 14 volts, amplifier 154D will not connect output 154H to ground and LED 154I will not turn on. Thus, battery life—ON/OFF indicator circuit 154 will cause LED 154I to blink at 2.3 second intervals during the 5-minute period when the current is on, providing the battery level is above 14 volts.

Polarity control circuit 156 comprises double-pole, double-throw switch 156A and 33 microfarad capacitor 156B. Capacitor 156B plays no essential part in the polarity control function, but is simply a filter capacitor which is connected between the ground and input line 149 and the positive voltage input 156C. One pole of switch 156A is connected to the positive 18 volt power supply through line 156C and the other pole is connected to the input line 152G from current control circuit 152. The upper terminal of the left throw and the lower terminal of the right throw of switch 156A are connected to output line 156D which goes to electrode 1. The lower terminal of the left throw and the upper terminal of the right throw of switch 156A are connected to output line 156E to electrode 2. Thus, if switch 156A is in the left hand throw position, electrode 1 will have positive polarity while electrode 2 will have a negative polarity and current will flow from electrode 1 to electrode 2. If the ionic substance is to be driven into the tissue is a positive ion, electrode 1 will be the active electrode when switch 156A is in the left hand position, while if the ionic substance is a negative ion, then electrode 2 will be the active electrode. When switch 156A is in the right hand throw position the polarity, direction of current flow of the electrodes is reversed.

In the embodiment shown in Fig. 12, switch 156A is a manual switch accessible from the exterior of the casing, such as 133 in Fig. 10, of the iontophoretic device. If appropriate markings are placed on the case, the position of the switch will provide an indication of the polarity of the electrodes. If the ionicity of the ionic substance is known, then the switch may also act as a means for indicating the active electrode.

An iontophoretic device employing the circuitry of Fig. 12 may be used by turning the device on, allowing the ionic substance from one electrode to be delivered, and then after the device turns itself off, manually reversing the polarity and turning the device on again so the second electrode will deliver its ionic substance. In this manner both electrodes may be used to deliver the ionic substance, and twice the amount of ionic substance can be delivered per application of the iontophoreric device.

Fig. 13 shows a detailed electronic diagram of another exemplary preferred embodiment of the invention. This is a more highly automated circuit than the circuit shown in Fig. 12 and includes polarity timing control circuit 160 which provides additional timing functions connected with the polarity switching, polarity switching circuit 162 which responds to signals from the timing circuits to automatically switch the polarity, active electrode indicator circuit 164 which automatically indicates which electrode is the active electrode, and ion type switch 166 which changes the polarity of the electrodes so that the active electrode indicator circuit 164 will properly indicate the active electrodes with both types of ionic substances.

The circuitry of Fig. 13 is the circuitry employed in embodiment of the iontophoretic generator shown in Figs. 9 and 10.

The portions of the circuitry of Fig. 13 not forming part of circuits 160, 162, 164 and 166 is the same as the circuitry just described in connection with Fig. 12, except for the portion associated with ripple counter 170B.

This latter portion differs from the circuit of Fig. 12 in that the value of capacitance 170G in the oscillator circuit 170A is chosen to be 0.1 microfarads and resistor 170H is selected so that the oscillation of circuit 170A will have a period of 73.2 milliseconds, and an additional output 170C is added to ripple counter 170B at the terminal of the thirteenth counter (No. 2 pin). In addition, output 170D which drives the LEDs in active electrode indicator circuit 164, is connected to the fifth output stage (No. 5 pin) rather than the sixth output stage. With these changes, output line 170C will become a logic "0" after switch 140 is turned on, then after a 5-minute period it will become a logic "1" and will remain at that state for a 5-minute period. Output line 170D will go to a logic "1" for a 1.15 second period every 2.3 seconds while the counter is running, just as the corresponding output 150R in Fig. 12. Output line 170E will become a logic "0" when the switch 140 is turned on and will go to a logic "1" approximately 10 minutes after the initiation of the counter. Thus the counter 170B in Fig. 13 will provide a signal through line 170C to the polarity timing control circuit that is a logic "0" for the first five minutes after the device is turned on and a logic "1" for the next five minutes. Output 170D will provide the same timing signal to the LED indicators in circuit 164 as the corresponding line 150R in Fig. 12. As can be seen from considering the discussion of the operation of NOR gates 150F, 150G and 150H in the discussion of Fig. 12, the signal from line 170E will cause NOR gate 170K to provide a signal to the polarity timing control circuit that becomes a logic "1" for a 10-minute period after switch 140 is turned on, and then becomes a logic "0".

Polarity timing control circuit 160 comprises AND gate 160A, NAND gate 160B, inverter 160C, monostable multivibrator 160D, capacitor 160E and resistor 160F. Multivibrator 160D is a conventional one-shot type circuit such as the RCA-CD4047A available from RCA Solid State Division, Box 3200, Summerville, NJ. 08876. Capacitor 160E is connected between pin 3 and pin 1 of multivibrator 160D and resistor 160F is connected between pin 3 and pin 2. The values of capacitor 160E and resistor 160F are chosen so as to provide an approximately 1 second decay period $(T_n)$ for the monostable vibrator 60D according to the formula

$$T_n = 2.48 \ RC \approx 1 \text{ second.}$$

Ripple counter output 170C is applied to the positive edge trigger input of monostable multivibrator 160D and the lower input of NAND gate 160B in polarity timing control circuit 160. The $\overline{Q}$ output of multivibrator 160D is applied to the upper input of NAND gate 160B and also to the upper input of AND gate 160A. The lower input of AND gate 160A is provided by the output of NOR gate 170K as discussed above. The output of NAND gate 160B is connected to one of the poles of switch 166 and is also provided as an input to inverter 160C. The output of inverter 160C is provided to the other of the poles of switch 166. The output of AND gate 160A is provided to the anode of constant current diode 172A which corresponds to the constant current diode 152A in the current control circuit of Fig. 12.

From the above connections it can be seen that when switch 140 is turned on a logic "0" signal is applied to NAND gate 160B. The output of NAND gate 160B will thus become a logic "1". The logic "0" signal from output 170C applied to the clock input of multivibrator 160D causes the $\overline{Q}$ output to become a logic "1". At the same time, the output of NOR gate 170K will become a logic "1", and thus both inputs to AND gate 160A will be a logic "11", causing its output to become a logic "1" immediately after switch 140 is turned on. This signal will initiate the ramp up of the current as discussed above in relation to Fig. 12. After a 5-minute period output line 170C goes to a logic "1", which causes the $\overline{Q}$ output of multivibrator 160D to become a logic "0". This logic "0" applied to AND gate 160A causes its output to become a logic "0" which causes the current to ramp down as discussed above in relation to Fig. 12. The logic "0" signal from the $\overline{Q}$ output of multivibrator 160D also is applied to the upper input of NAND gate 160B which causes its output to remain at logic "1". After a 1-second interval, after which the current in the current control circuit is fully ramped down to zero, monostable multivibrator 160D times out and the $\overline{Q}$ output returns to a logic "1". Now, since both inputs to NAND gate 160B are now a logic "1" its output becomes a logic "0" which switches the polarity of the electrodes, in a manner which shall be described below in the discussion of circuit 162. At the same time the logic "1" signal from the $\overline{Q}$ output of the multivibrator 160D causes the output of AND gate 160A to return to a logic "1" which again causes the current to ramp up to its full value. After another 5-minute interval (10 minutes after the device was turned on) the output of NOR gate 170K becomes a logic "0" which causes the output of AND gate 160A to become a logic "0" which again ramps down the current. At the same time the logic "1" on output 170E of counter 170B causes the counter to be reset which turns the counter and indicators off as discussed in relation to Fig. 12.

Ion switch 166 consists of a double-pole, double-throw switch. Its right and left hand poles are connected to outputs of NAND gate 160B and inverter 160C as discussed above. Its upper left throw and lower right throw are connected to output line 166A, while its lower left throw and upper right throw are connected to output line 166B. Because of inverter 160C, the poles of switch 166 will always be in opposite logic states when the device is operating. Thus, output lines 166A and 166B will also be in opposite logic states, whether the switch 166 is in its upper throw position or its lower throw position. As can be seen from the connections discussed above, the action of the switch will be to reverse the signals on lines 166A and 166B.

Polarity switching circuit 162 comprises FETs 162A, 162B, 162C and 162D and 133 microfarad capacitor 162E. Capacitor 162E does not perform an active role in the polarity switching circuit, but is a filter capacitor which is connected and functions as the corresponding capacitor 156B in Fig. 12. The gates of FETs 162A and 162B are connected to the output line 166B from ion type switch 166. The gates of FETs 162C and 162D are connected to output line 166A from the same switch. The source of FET 162A is connected to the output line 172B from the current control circuit, while the drain is connected to the output to electrode No. 2(124). The source of FET 162B is connected to the output to electrode No. 1(125) while its drain is connected to the +18 volt power supply. The drain of FET 162C is also connected to the +18 volt power supply while its source is connected to the output line to electrode No. 124. The drain of FET 162D is connected to the output line to electrode No. 125, while its source is connected to the current output line 172B.

If ion type switch 166 is in the upper (positive) throw position, then during the first 5 minutes of operation of the device, line 166A will be in a logic "1" state while line 166B will be in a logic "0" state. The logic "0" signal applied to the gates of FETs 162A and 162B will keep these FETs turned off, while the logic "1" signal applied to the gates of FETs 162C and 162D will turn these FETs on. Current will thus flow from the +18 volt voltage source through FET 162C to electrode No. 2(124). If the electrodes are applied to tissue the current will flow through the tissue and then through electrode 1(125) and FET 162D back through current input line 172B. Thus, if ion type switch is in the positive position, which means the ionic substance is positive, electrode 124 will be the active electrode and electrode 125 will be the negative electrode during the first 5-minute cycle. After the first five minutes, the signal from NAND gate 160B will reverse, and the signals from lines 166A and 166B will also reverse, causing FETs 162A and 162B to turn on and FETs 162D and 162C to turn off. Current will now flow from positive input 62F through FET 162B to electrode 125, through the tissue and to electrode 124, and through FET 162A to current line 172B. Thus, during this 5-minute period, electrode 125 will be the active electrode. If the ionic substance is negative, ion type switch will be placed in the down (negative) position and the directions of the currents will be reversed in the two cycles. Since the sign of the ionic substance is also reversed, electrode 124 will still be the active electrode during the first cycle and electrode 125 will still be the active electrode during the second cycle.

Turning attention to the active electrode indicator circuit 164, this circuit comprises inverter 164A, AND gates 164B and 164C, 100 kilohm resistors 164D and 164E, No. 2N4338 FETs 164F and 164G, 1.5 kilohm resistors 164H and 164I, and LEDs 130 and 131. Line 170D from counter 170B is connected to one of the inputs of gates 164B and 164C. Output 170C from counter 170B is connected to one of the inputs of gate 164C and through inverter 164A to one of the inputs of gate 164B. The output of gate 164B is connected through resistor 164D to the gate of FET 164F, while the output of gate 164C is connected through resistor 164E to the gate of FET 164G. The drains of FETs 164F and 164G are connected to the +18 volt power source. The source of FET 164F is connected through resistor 164H and LED 130 to the output (No. 6 pin) of amplifier 174A. Likewise, the source of FET 164G is connected through resistor 164I and LED 131 to the output of amplifier 174A.

As can be seen from the connections above, immediately after switch 140 is turned on line 170C will be a logic "0" and thus the right input to AND gate 164B will be logic "1" while the right input to AND gate 164C will be a logic "0". The logic "0" signal on AND gate 164C will hold its output to a logic "0", thus holding FET 164G in the off condition. As counter 170B begins to count, line 170D will go to a logic "1" and return to a logic "0" over a 2.3 second period as discussed above. During the time it is a logic "1" both the inputs to AND gate 164B will be a logic "1" and thus, its output will go to a logic "1", turning FET 164F on and allowing current to flow through LED 130, as was described earlier in connection with Fig. 12. At the times in which line 170D goes to a logic "0" the output of gate 164B will return to a logic "0" turning off FET 164F and LED 130. Thus, during the first 5-minute cycle, LED 130 will blink at the rate discussed above. During the second 5-minute cycle line 170C goes to a logic "1" which holds the output of gate 164B at a logic "0" state holding FET 164F off, at the same time permitting the output from gate 164C to oscillate between the logic "0" and logic "1" states, alternately turning on and off FET 164G, causing LED 131 to alternately turn on and off. Thus, during the second 5-minute cycle LED 131 will blink. As can be seen from comparing the operation of the polarity switching circuit 162 and ion type switch 166 discussed above, LED 130 will blink while electrode 124 is the active electrode, while LED 131 will blink while electrode 125 is the active electrode.

There has been described a novel apparatus that greatly simplifies the administration of drugs and other chemicals by the iontophoretic method, and thereby makes the iontophoretic process a much more practical method of administration of such substances. While the invention has been described above in connection with particular embodiments, one skilled in the art will appreciate that the invention is not necessarily so limited and that numerous other embodiments and departures from the embodiments may be made without departing from the invention concepts. For example many other equivalent electronic circuits may be substituted for the circuits described. Within the circuits, many substitutions and additions can be made. For example, different application times can be chosen by altering the oscillation circuit consisting of resistors 80I, 80J, 80L and capacitor 80K, or by substituting different counters for ripple counter 80M. The time may be made selectable by substituting a variable resistance for resistor 80I. Likewise, the other oscillator circuits shown may be varied. Many different currents and voltages can be selected by altering the current control circuit for example, the resistor 82F can be replaced by a potentiometer so that current applied can be externally varied. Other more complex timer control and current control circuits can be introduced to permit more variability in any given instrument as regards to the control of dosage and the rate and time of administration of the dosage. The electronics described have been discrete electronic components, but it is evident that equivalent microelectronic chips can be designed which would greatly reduce the size and power requirements of the element and thus permit reduction in size of the entire system or microprocessors can be incorporated for functions such as automatic power up, power down, controlling daily dosages, etc. Switching means such as 156A, 166 and 162 may be replaced with other types of switching means. Likewise, housing 10 or 132 can take on many configurations and sizes. For example, the housing may be made in the form of a flexible belt of plastic or other insulative material containing the circuits in microelectronics form and the plates 20 and 21 or 126 and 127 may be in the form of metal layers painted, evaporated upon, or otherwise attached to the plastic belt. Plates 20 and 21 or 126 and 127 need not necessarily be square but may be almost any shape, and may be formed to conform to particular body contours. The plates need not be of the same size, but one plate may be substantially larger than the other. In some embodiments both plates may be relatively small compared to the housing rather than substantially covering the entire underside of the housing as in the embodiments shown. The plates need not be made of stainless steel, but may be made of other metal or any other conducting materials including flexible conducting materials. Many additional features, controls and gadgets can be added to the electronic circuitry and housing while still employing the inventive elements. For example, instead of using two LEDs to indicate the two cycles, one LED could be used, and it could be made to blink at different rates for each of the cycles. Or, alternatively, many other indicating means could be used. Many other equivalent electronic circuits may be substituted for the circuits described, and within the circuits many substitutions and additions can be made. For example, resistor 154A could be eliminated and a larger resistor value could be substituted for resistor 154E to form an equivalent circuit. Many additional features, and controls can be added to the electronic circuitry while still employing the inventive elements. Those skilled in the art will also see many other variations of the invention.

**Claims**

1. A device for use in iontophoretically introducing an ionic substance into body tissue, comprising a casing (10, 132) containing an electrical current source (11, 110) and active electrode means mounted on said casing and electrically coupled to said current source, said active electrode means including first electrode plate means (20, 126) located on said casing for mounting on said casing an electrode element (35, 124) containing said ionic substance and for electrically coupling said electrode element to said current source; and further comprising indifferent electrode means electrically coupled to said current source, characterized in that said casing (10, 132) is insulating; that said indifferent electrode means likewise is mounted on said casing and includes second plate means (21, 127) located on said casing (10, 132) for mounting on said casing a second electrode element (28A, 125) and for electrically coupling said second element to said electric current source; and that said electrode elements are removably provided on said plate means in the form of adhesive strips.

2. A device as described in claim 1, wherein said first electrode plate means (20, 126) and said second electrode plate means (21, 127) each comprise a stainless steel plate.

3. A device as described in claim 1 or 2 and further comprising a means (50, 143) extending between said first and second electrode plates (20, 21; 126, 127) for preventing electrical contact between conductive elements applied to said plates.

4. A device as described in claim 3 wherein said means extending between said plates (20, 21; 126, 127) comprises a raised portion (50, 143) of said casing (10, 132).

5. A device as described in any one of claims 1 to 4 and further comprising means for distinguishing said first electrode plate means (20, 126) from said second electrode plate means (21, 127) so that it may be quickly and easily determined which of the two electrode plate means should receive a first electrode element (35, 124) and which of the two electrode means should receive a second electrode element (28A, 125).

6. A device as described in any one of claims 1 to 5 and further comprising a self-adhering strap (55, 144) mounted on said casing (10, 132) for assisting in securing said casing to said body.

7. A device as described in any one of claims 1 to 6 wherein said electric current source (11, 110) includes a battery (12, 120) and further comprising a means (61, 130, 131) for indicating whether or not said battery has sufficient power remaining for satisfactory operation of said device.

8. A device as described in any one of claims 1 to 7 and further comprising means (133, 156, 162) for switching the polarity of said electrode.

9. A device as described in claim 8 wherein said means for switching comprising a digital electronic switching circuit (156, 162).

10. A device as described in claim 8 or 9 and further including means (152) for ramping down said current prior to the switching of polarity and for ramping up said current after the switching of polarity.

11. A device as described in any one of claims 8 to 10 and further including a means (130, 131, 133, 156A) for indicating the polarity of the electrodes.

12. A device as described in any one of claims 8 to 11 and further comprising: timing means (160) for providing at least one timing signal; and wherein said means for switching (162) comprises a means responsive to said timing signal for switching the polarity at a preselected time.

13. A device as described in claim 12 and further comprising means (172A) responsive to a signal generated by the timing means (160) for ramping down the current prior to said time at which said polarity is switched and for ramping up said current after said polarity is switched.

14. A device as described in any one of claims 8 to 13 and further including a means (164) for indicating which of said electrodes (124, 125) is delivering said ionic substance.

15. A device as described in claim 14 and further including a ion switch means (166) for correlating said indication with the electric charge type of said ionic substance.

**Revendications**

1. Dispositif destiné à introduire par iono-phorèse une substance ionique dans des tissus du corps, comprenant un boitier (10, 132) qui contient une source de courant électrique (11, 110) et une électrode active montée sur ledit boitier et connectée électriquement à ladite source de courant, ladite électrode active comprenant une première plaque d'électrode (20, 126) disposée sur ledit boitier pour le montage sur ce boitier d'un élément d'électrode (35, 124) qui contient ladite substance ionique, et pour connecter électriquement ledit élément d'électrode à ladite source de courant; et comportant en outre une électrode indifférente connectée électriquement à ladite source de courant, caractérisé en ce que ledit boitier (10, 132) est isolant; en ce que ladite électrode indifférente est également montée sur le dit boitier et comporte une seconde plaque (21, 127) disposée sur ledit boitier (10, 132) pour le montage sur ce boitier d'un second élément d'électrode (28A, 125) et pour connecter électriquement ledit second élément à ladite source de courant électrique; et en ce que lesdits éléments d'électrode sont prévus de façon amovible sur lesdites plaques sous forme de bandes adhésives.

2. Dispositif selon la revendication 1, dans lequel ladite première plaque d'électrode (20, 126) et ladite second plaque d'électrode (21, 127) consistent chacune en une plaque d'acier inoxydable.

3. Dispositif selon la revendication 1 ou 2, comportant en outre un dispositif (50, 143) disposé entre ladite première et ladite second plaques d'électrodes (20, 21; 126, 127) pour éviter tout contact électrique entre des éléments conducteurs appliqués sur lesdites plaques.

4. Dispositif selon la revendication 3, dans lequel ledit dispositif disposé entre lesdites plaques (20, 21; 126, 127) consiste en une partie en relief (50, 143) dudit boitier (10, 132).

5. Dispositif selon l'une quelconque des revendications 1 à 4, comportant en outre un dispositif pour distinguer ladite première plaque d'électrode (20, 126) de ladite seconde plaque d'électrode (21, 127) de manière qu'il puisse être déterminé rapidement et facilement celle des deux plaques d'électrodes qui doit recevoir un premier élément d'électrode (35, 124) et celle des deux électrodes qui doit recevoir un second élément d'électrode (28A, 125).

6. Dispositif selon l'une quelconque des revendications 1 à 5, et comportant en outre une bande auto-collante (55, 144) montée sur ledit boitier (10, 132) pour aider à fixer ledit boitier sur ledit corps.

7. Dispositif selon l'une quelconque des revendications 1 à 6, dans lequel ladite source de courant électrique (11, 110) comporte une batterie (12, 120) et comprenant en outre un dispositif (61, 130, 131) pour indiquer si ladite batteire a ou non une puissange suffisante qui subsiste pour un fonctionnement satisfaisant dudit dispositif.

8. Dispositif selon l'une quelconque des revendications 1 à 7, et comportant en outre un dispositif (133, 156, 162) pour commuter la polarité de ladite électrode.

9. Dispositif selon la revendication 8, dans lequel ledit dispositif de commutation consiste en un circuit de commutation électronique numérique (156, 162).

10. Dispositif selon la revendication 8 ou 9, et comportant en outre un dispositif (152) pour faire décroître ledit courant avant la commutation de polarité et pour faire croître ledit courant après la commutation de polarité.

11. Dispositif selon l'une quelconque des revendications 8 à 10, et comportant en outre un dispositif (130, 131, 133, 156A) pour indiquer la polarité des électrodes.

12. Dispositif selon l'une quelconque des revendications 8 à 11, et comportant en outre un dispositif de temporisation (160) pour produire an moins un signal de temporisation; et dans lequel ledit dispositif de commutation (162) consiste en un dispositif qui réagit audit signal de temporisation pour commuter la polarité à un instant prédéterminé.

13. Dispositif selon la revendication 12, et comportant en outer un dispositif (172A) réagissant à un signal produit par le dispositif de temporisation (160) en faisant décroître le courant avant ledit instant auquel ladite polarité est commutée et pour faire croître ledit courant après que la polarité a été commutée.

14. Dispositif selon l'une quelconque des revendications 8 à 13, et comportant en outre un dispositif (164) pour indiquer celle des desdites électrodes (124, 125) qui délivre ladite substance ionique.

15. Dispositif selon la revendication 14, et comportant en outre un dispositif de commutation d'ions (166) pour mettre en corrélation ladite indication avec le type de charge électrique de ladite substance ionique.

**Patentansprüche**

1. Gerät zur Verwendung bein iontophoretischen Einbringen einer ionischen Substanz in Körpergewebe mit einem eine elektrische Stromquelle (11, 110) enthaltenden Gehäuse (10, 132) und einer aktiven Elektrodenanordnung, die auf dem Gehäuse angebracht und mit der Stromquelle elektrisch gekoppelt ist, wobei die aktive Elektrodenanordnung eine auf dem Gehäuse befindliche erste Elektrodenplattenanordnung (20, 126) zum Anbringen eines die ionische Substanz enthaltenden Elektrodenelements (35, 124) auf dem Gehäuse und zum elektrischen Ankoppeln des Elektrodenelements an die Stromquelle aufweist, sowie mit einer indifferenten Elektrodenanordnung, die an die Stromquelle elektrisch angekoppelt ist, dadurch gekennzeichnet, daß das Gehäuse (10, 132) isolierend ist; daß die indifferente Elektrodenanordnung gleichfalls auf dem Gehäuse angebracht ist und eine auf dem Gehäuse (10, 132) befindliche zweite Plattenanordnung (21, 127) zum Anbringen eines zweiten Elektrodenelements (28A, 125) auf dem Gehäuse und zum elektrischen Ankoppeln des zweiten Elements an die elektrische Stromquelle aufweist; und daß die Elektrodenelements auf den Plattenanordnungen in Form von Klebestreifen lösbar angeordnet sind.

2. Gerät nach Anspruch 1, wobei die erste Elektrodenplattenanordnung (20, 126) und die zweite Elektrodenplattenanordnung (21, 127) jeweils eine Platte aus rostfreiem Stahl aufweisen.

3. Gerät nach Anspruch 1 oder 2, das ferner mit einer sich zwischen den ersten und zweiten Elektrodenplatten (20, 21; 126, 127) erstreckenden Anordnung (50, 143) zur Verhindern eines elektrischen Kontakts zwischen auf die Platten aufgebrachten leitenden Elementen versehen ist.

4. Gerät nach Anspruch 3, wobei die sich zwischen den Platten (20, 21; 126, 127) erstreckende Anordnung einen hochstehenden Teil (50, 143) des Gehäuses (10, 132) aufweist.

5. Gerät nach einem der Ansprüche 1 bis 4 mit Mitteln zum Unterscheiden der ersten Elektrodenplattenanordnung (20, 126) von der zweiten Elektrodenplattenanordnung (21, 127), so daß rasch und leicht bestimmt werden kann, welche der beiden Elektrodenplattenanordnungen ein erstes Elektrodenelement (35, 124) und welche der beiden Elektrodenanordnungen ein zweites Elektrodenelement (28A, 125) aufnehmen soll.

6. Gerät nach einem der Ansprüche 1 bis 5 mit einem auf dem Gehäuse (10, 132) angeordneten selbsthaftenden Band (55, 144) zum Befestigen des Gehäuses an dem Körper.

7. Gerät nach einem der Ansprüche 1 bis 6, bei dem die elektrische Stromquelle (11, 110) eine Batterie (12, 120) aufweist und das ferner mit Mitteln (61, 130, 131) zur Anzeige, ob die Batterie für einen befriedigenden Betrieb des Gerätes ausreichende verbliebene Energie hot oder nicht, versehen ist.

8. Gerät nach einem der Ansprüche 1 bis 7 mit Mitteln (133, 156, 162) zum Umschalten der Polarität der Elektrode.

9. Gerät nach Anspruch 8, wobei die Mittel zum Umschalten einen digitalen elektronischen Schaltkreis (156, 162) aufweisen.

10. Gerät nach Anspruch 8 oder 9 mit Mitteln (152) zum allmählichen Absenken des Stromes vor dem Umschalten der Polarität und zum allmählichen Erhöhen des Stromes nach dem Umschalten der Polarität.

11. Gerät nach einem der Ansprüche 8 bis 10 mit einer Anordnung (130, 131, 133, 156A) zum Anzeigen der Polarität der Elektroden.

12. Gerät nach einem der Ansprüche 8 bis 11 mit einer Zeitgeberanordnung (160) zum Anliefern mindestens eines Zeitgabesignals, wobei die Umschaltmittel (162) eine auf das Zeitgabesignal ansprechende Anordnung zum Umschalten der Polarität zu einer vorgewählten Zeit aufweisen.

13. Gerät nach Anspruch 12 mit einer auf das von der Zeitgeberanordnung (160) erzeugte Signal ansprechenden Anordnung (172A) zum allmählichen Absenken des Stroms vor dem Zeitpunkt der Umschaltung der Polarität und zum allmählichen Erhöhen des Stromes nach dem Umschalten der Polarität.

14. Gerät nach einem der Ansprüche 8 bis 13 mit einer Anordnung (164) zur Anzeige welche der Elektroden (124, 125) die ionische Substanz abgibt.

15. Gerät nach Anspruch 14 mit einer Ionenschalteinrichtung (166) zum Korrelieren der Anzeige mit dem elektrischen Ladungstyp der ionischen Substanz.

FIG. 1

FIG. 2A

FIG. 2B

FIG. 3

FIG. 4

FIG. 5

| CURRENT SOURCE | | ACTIVE ELECTRODE |
| INDIFFERENT ELECTRODE |

FIG. 6

| TIMER CONTROL | CURRENT CONTROL | ACTIVE ELECTRODE |
| INDIFFERENT ELECTRODE |
| BATTERY LIFE— ON/OFF INDICATOR |

1

FIG. 7

FIG. 8

FIG. 9

FIG. 10

FIG. 11

3

FIG. 12

FIG. 13

ACTIVE ELECTRODE INDICATOR

POLARITY TIMING CONTROL

POLARITY SWITCHING

RIPPLE COUNTER

ION TYPE SWITCH